# DEMANDE DE BREVET EUROPEEN

(11) **EP 1 245 225 A1**
(43) Date de publication de la demande: **02.10.2002**
(21) Numéro de dépôt: 02290685.3
(22) Date de dépôt: 19.03.2002
(51) Int. Cl.: A61K 7/50

(54) **Compositions cosmétiques détergentes contenant un tensioactif anionique dérivé d'acides aminés**

(30) Priorité: 30.03.2001 FR 0104381
(71) Demandeur: L'OREAL, 75008 Paris (FR)
(72) Inventeur: Perron, Béatrice, 78350 Jouy en Josas (FR); Restle, Serge, 95390 Saint-Prix (FR)
(74) Mandataire: Le Blainvaux Bellegarde, Françoise

(57) **Abrégé**

L'invention concerne de nouvelles compositions cosmétiques détergentes comprenant dans un milieu cosmétiquement acceptable au moins un tensioactif anionique choisi parmi les composés N-acylés d'aminoacides polycarboxyliques mono ou poly amidifiés et leurs sels, et au moins un agent conditionneur particulier.

Cette association apporte des propriétés cosmétiques (lissage, légèreté, douceur) sans phénomène de regraissage des fibres kératiniques.

Ces compositions sont utilisées notamment pour le lavage et/ou le conditionnement des matières kératiniques telles que les cheveux ou la peau.

## Description

La présente invention concerne de nouvelles compositions cosmétiques comprenant dans un milieu cosmétiquement au moins un tensioactif anionique choisi parmi les composés N-acylés d'aminoacides polycarboxyliques mono ou poly amidifiés et leurs sels et au moins un agent conditionneur insoluble dans l'eau particulier.

Pour le nettoyage et/ou le lavage des cheveux et/ou de la peau, l'utilisation de compositions détergentes (shampooing ou gel-douche) à base essentiellement d'agents tensioactifs classiques de type notamment anionique, non ionique et/ou amphotère, mais plus particulièrement de type anionique, est courante. Ces compositions sont appliquées sur cheveux ou peau mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur les matières kératiniques des dommages plus ou moins marqués liés en particulier à l'élimination progressive des lipides ou protéines contenues dans ou à la surface de ces dernières.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes.

Toutefois, et malgré les progrès réalisés récemment dans le domaine des shampooings à base de polymères cationiques et/ou de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

Les tensioactifs anioniques N-acylés d'aminoacides polycarboxyliques mono ou poly amidifiés et leurs sels ont déjà été préconisés dans des compositions cosmétiques détergentes. Ils ont été décrits par exemple dans la demande de brevet WO97/03171.

Les compositions de lavage des cheveux utilisant ces tensioactifs seuls ne conduisent pas à de propriétés cosmétiques satisfaisantes.

L'invention a donc pour but de proposer des compositions cosmétiques en particulier détergentes présentant des propriétés cosmétiques améliorées, en particulier le démêlage, le lissage et la douceur des cheveux.

Or, la demanderesse a maintenant trouvé que l'association d'agents conditionneurs insolubles particuliers et de tensioactifs anioniques N-acylés d'aminoacides polycarboxyliques mono ou poly amidifiés et leurs sels permettait d'atteindre ces buts.

Ces nouvelles compositions permettent de mieux déposer ces agents conditionneurs insolubles sur les matières kératiniques (notamment les cheveux) qu'une composition contenant des tensioactifs anioniques classiques tels que les sels d'acide N-cocoylglutamique.

Les compositions conformes à l'invention confèrent aux matières kératiniques notamment les cheveux, un remarquable effet traitant qui se manifeste notamment par une facilité de démêlage, ainsi qu'un apport de légèreté, de lissage, de douceur et de souplesse sans aucune sensation de toucher chargé.

L'invention a ainsi pour objet une composition cosmétique en particulier détergente, caractérisée en ce qu'elle comprend, dans un milieu cosmétiquement acceptable, au moins un tensioactif anionique choisi parmi les composés N-acylés d'aminoacides polycarboxyliques mono ou poly amidifiés et leurs sels et au moins un agent conditionneur insoluble choisi parmi :
- les huiles de synthèse
- les huiles animales ou végétales et
- les huiles fluorées ou perfluorées,
- les cires naturelles ou synthétiques
- les composés de type céramides
- les esters d'acides carboxyliques choisis parmi les esters de monoalcools et les esters de polyols, les polyols ayant au moins 3 atomes de carbone.

Un autre objet de l'invention concerne l'utilisation d'au moins un tensioactif anionique choisi parmi les composés N-acylés d'aminoacides polycarboxyliques mono ou poly amidifiés et leurs sels dans, ou pour la fabrication d'une composition cosmétique comprenant au moins un agent conditionneur insoluble tel que défini ci-dessus.

Un autre objet de l'invention concerne un procédé de traitement des matières kératiniques, telles que les cheveux, caractérisé en ce qu'il consiste à appliquer sur lesdites matières des compositions cosmétiques selon l'invention.

L'invention a encore pour objet l'utilisation d'une composition selon l'invention pour améliorer le démêlage ou le lissage des cheveux, ou pour apporter du volume, de la légèreté, de la douceur, de la souplesse ou de la discipline aux cheveux.

Selon la présente invention, par matières kératiniques, on comprend les cheveux, les cils, les sourcils, la peau, les ongles, les muqueuses ou le cuir chevelu et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Les tensioactifs anioniques N-acylés d'aminoacides polycarboxyliques mono ou poly amidifiés et leurls peuvent avoir la formule suivante (I): 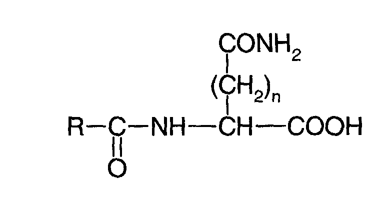 dans laquelle :
R désigne un radical hydrocarboné, saturé ou insaturé, linéaire ou ramifié comportant de 5 à 29 atomes de carbone.
R désigne de préférence un radical alkyle ou alkényle mono ou polyinsaturé comportant de 5 à 29 atomes de carbone et de préférence de 7 à 22 atomes de carbone.
n est égal à 1 ou 2.

Des composés N-acylés d'aminoacides polycarboxyliques mono ou poly amidifiés préférés selon la présente invention sont des composés de formule (I) dans laquelle R désigne plus particulièrement un radical alkyle saturé, linéaire ou ramifié comportant de 7 à 29 atomes de carbone, de préférence de 7 à 22 atomes de carbone.

Les sels des composés de formule (I) peuvent être des sels de métaux alcalins (par exemple sodium ou potassium), de métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Parmi les tensioactifs de formule (I), on peut citer plus particulièrement les sels de N-cocoylglutamine et notamment le sel de triéthanolamine de N-cocoylglutamine tel que le produit commercialisé sous la dénomination FOAM UP DOUCE GM par la société KYOWA HAKKO.

Selon l'invention, le ou les tensioactifs anioniques choisis parmi les composés N-acylés d'aminoacides polycarboxyliques mono ou poly amidifiés et leurs sels peuvent représenter de 1 % à 30 % en poids, de préférence de 3 % à 15 % en poids par rapport au poids total de la composition finale.

Dans le cadre de la présente demande, on entend par agent conditionneur tout agent ayant pour fonction l'amélioration des propriétés cosmétiques des matières kératiniques telles que les cheveux, en particulier la douceur, le démêlage, le toucher, l'électricité statique.

Les agents conditionneurs insolubles conformément à l'invention peuvent être solides, liquides ou pâteux à température ambiante (25°C) et à pression atmosphérique, ils peuvent notamment se présenter sous forme d'huiles, de cires, de résines ou de gommes.

Les agents conditionneurs insolubles sont notamment dispersés dans les compositions sous forme de particules ayant généralement une taille moyenne en nombre allant de 2 nanomètres à 100 microns, de préférence allant de 30 nanomètres à 20 microns (mesurée avec un granulomètre).

Les agents conditionneurs insolubles dans l'eau sont insolubles dans l'eau à une concentration supérieure ou égale à 0,1% en poids dans l'eau à 25°C, c'est à dire qu'ils ne forment pas une solution macroscopiquement isotrope transparente.

Les huiles de synthèse sont notamment les polyoléfines en particulier les poly-α-oléfines et plus particulièrement :
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence allant de 1000 à 15000.
   A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.
   De tels produits sont vendus par exemple sous les dénominations ETHYLFLO par la société ETHYL CORP., et d'ARLAMOL PAO par la société ICI.

Les huiles animales ou végétales sont choisies préférentiellement dans le groupe formé par les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone en particulier alkyle ou alkényle, par exemple, l'huile de Purcellin ou la cire liquide de jojoba ;
On peut également utiliser les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre 1983, pp. 30-33.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Selon la présente invention, les composés de type céramide sont notamment les céramides et/ou les glycocéramides et/ou les pseudocéramides et/ou les néocéramides, naturelles ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

Les composés de type céramide utilisables selon la présente invention répondent préférentiellement à la formule générale (IV): 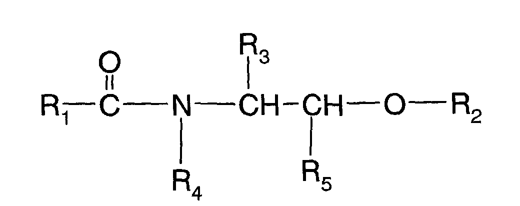 dans laquelle :
- R₁ désigne :
   - soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifié par un acide gras saturé ou insaturé, linéaire ou ramifié, éventuellement mono ou polyhydroxylé, en C₁-C₃₅;
   - soit un radical R"-(NR°-CO)-R', R° désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone va de 9 à 30, R' étant un radical divalent.
   - soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, mono ou polyhydroxylé ou non hydroxylé, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ; de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)_{m,} sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

Parmi les composés de formule (IV), on préfère les céramides et/ou glycocéramides dont la structure est décrite par DOWNING dans Journal of Lipid Research Vol. 35, 2060-2068, 1994, ou ceux décrits dans la demande de brevet français FR-2 673 179, dont les enseignements sont ici inclus à titre de référence.

Les composés de type céramide plus particulièrement préférés selon l'invention sont les composés de formule (IV) pour lesquels R₁ désigne un alkyle saturé ou insaturé dérivé d'acides gras en C₁₄-C₂₂ éventuellement hydroxylé; R₂ désigne un atome d'hydrogène ; et R₃ désigne un radical linéaire en C₁₁₋₁₇ éventuellement hydroxylé et de préférence en C₁₃₋₁₅.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine
ou les mélanges de ces composés.

Les huiles fluorées ou perfluorées sont par exemple les perfluoropolyéthers décrits notamment dans la demande de brevet EP-A-486135 et les composés fluorohydrocarbonées décrites notamment dans la demande de brevet WO 93/11103. L'enseignement de ces deux demandes est totalement inclus dans la présente demande à titre de référence.

Le terme de composés fluorohydrocarbonés désigne des composés dont la structure chimique comporte un squelette carboné dont certains atomes d'hydrogène ont été substitués par des atomes de fluor.

Les huiles fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers ;

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Selon l'invention, les esters peuvent être de préférence liquides à une température inférieure ou égale à 30 °C.

Les esters de monoalcools sont en particulier des esters d'acides mono, di, tri ou tétracarboxyliques de préférence en C₂-C₂₆ et de monoalcool de préférence en C₁-C₂₆, le nombre total de carbone des esters étant de préférence supérieur ou égal à 10 et inférieur à 80.

Les esters d'acides monocarboxyliques sont notamment les monoesters d'acides aliphatiques saturés ou insaturés, linéaires ou ramifiés en C₂-C₂₆ et de monoalcools aliphatiques saturés ou insaturés, linéaires ou ramifiés en C₁-C₂₆ , le nombre total de carbone des esters étant supérieur ou égal à 10.

Parmi ces monoesters , on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en C4-C22 et d'alcools en C1-C22.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle; le stéarate d'octyldodécyl stéaroyl ; l'érucate de tridécyle ; le citrate de triisopropyle ; le citrate de triisotéaryle ; le citrate de trioctyldodécyle ; le citrate de trioléyle.

Les esters de polyols ayant au moins 3 atomes de carbone sont notamment choisis parmi les esters d'acides mono di ou tricarboxyliques de préférence en C2-C26 et d'alcools di, tri, tétra ou pentahydroxy en C3-C26.
On peut notamment citer : l'undecylénate de glycéryle; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate ou le dicaprate de propylène glycol ; trilactate de glycéryle ; trioctanoate de glycéryle.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de 2-octyldodécyle, le stéarate de butyle, le stéarate d'isobutyle ; le malate de dioctyle, le laurate d'hexyle, l'isononanate d'isononyle et l'octanoate de cétyle.

Selon l'invention, le ou les agents conditionneurs insolubles selon l'invention peuvent représenter de 0,001 % à 20 % en poids, de préférence de 0,01 % à 10% en poids et plus particulièrement de 0,1 à 3% en poids par rapport au poids total de la composition finale.

Les compositions de l'invention contiennent en outre avantageusement au moins un autre agent tensioactif qui est généralement présent en une quantité allant de 0,1% à 40% en poids environ, de préférence allant de 3% à 30% et encore plus préférentiellement allant de 5% à 20%, par rapport au poids total de la composition.

Cet agent tensioactif peut être choisi parmi les agents tensioactifs anioniques, amphotères, non-ioniques ou leurs mélanges.

Les tensioactifs additionnels convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersulfates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères additionnels, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂- CHOH - SO3H
R₅ désigne un radical alkyle d'un acide carboxylique présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 7ème édition, 1997, sous les dénominations Disodium Cocoamphodiacetate, Disodium Lauroamphodiacetate, Disodium Capryloamphodiacetate, Disodium Caproamphodiacetate, Disodium Cocoamphodipropionate, Disodium Lauroamphodipropionate, Disodium Caproamphodipropionate, Disodium Capryloamphodipropionate, Lauroamphodipropionic acid, Cocoamphodipropionic acid.
A titre d'exemple on peut citer le disodium cocoamphodiacetate commercialisé sous la dénomination commerciale MIRANOL® C2M concentré par la société RHODIA CHIMIE.

Dans les compositions conformes à l'invention, on utilise de préférence des mélanges d'agents tensioactifs et en particulier des mélanges d'agents tensioactifs anioniques ou des mélanges d'agents tensioactifs anioniques et d'agents tensioactifs amphotères ou non ioniques.

On utilise de préférence comme agent tensioactif anionique additionnel les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélanges avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un agent tensioactif amphotère tel que les alkylbétaïnes en particulier la cocobétaïne commercialisée sous la dénomination "DEHYTON® AB 30" en solution aqueuse à 32 % de MA par la société HENKEL ou tel que les alkyl (C₈-C₂₀) amidoalkyl (C₁-C₆) bétaïnes en particulier la TEGOBETAINE® F 50 commercialisée par la société GOLDSCHMIDT.

Le(s) agent(s) tensioactif(s) anionique(s) différents composés N-acylés d'aminoacides polycarboxyliques mono ou poly amidifiés et leurs sels sont généralement présents à raison de 1 à 30 % en poids, de préférence de 3 à 15 % en poids, par rapport au poids total de la composition.

Le(s) agent(s) tensioactif(s) amphotère(s) ou non ioniques sont généralement présents à raison de 0,5 à environ 15% en poids, de préférence de 1 à 5% en poids, par rapport au poids total de la composition.

La quantité et la qualité des tensioactifs sont celles suffisantes pour conférer à la composition finale un pouvoir moussant et/ou détergent satisfaisant.

Dans la composition selon la présente invention, la totalité des tensioactifs détergents représente généralement de 4 à 50% en poids et de préférence de 6 à 35% en poids et plus particulièrement de 8 à 25% en poids par rapport au poids total de la composition.

La composition de l'invention peut également contenir au moins un additif choisi parmi les épaississants, les parfums, les agents nacrants, les conservateurs, les filtres solaires, les silicones, les polymères anioniques ou non ioniques ou amphotères, les polymères cationiques, les protéines, les hydrolysats de protéines, les acides gras à chaînes linéaires ou ramifiées en C₁₆-C₄₀ tels que l'acide méthyl-18 eicosanoique, les hydroxyacides, les vitamines, les provitamines telles que le panthénol, les agents antipelliculaires et tout autre additif classiquement utilisé dans le domaine cosmétique qui n'affecte pas la stabilité et les propriétés des compositions selon l'invention.

Ces additifs sont éventuellement présents dans la composition selon l'invention dans des proportions pouvant aller de 0,001 à 50% en poids par rapport au poids total de la composition. La quantité précise de chaque additif est déterminée facilement par l'homme du métier selon sa nature et sa fonction.

Le milieu cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols, les polyols tels que la glycérine.
De préférence, la composition comprend de 50 à 95 % en poids d'eau par rapport au poids total de la composition.

Les compositions détergentes selon l'invention présentent un pH final généralement allant de 3 à 10. De préférence, ce pH va de 4 à 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions selon l'invention peuvent contenir également des synergistes de mousses tels que des 1,2-alcanediols en C₁₀-C₁₈ ou des alcanolamides gras dérivés de mono ou de diéthanolamine.

Les compositions conformes à l'invention peuvent être utilisées pour le lavage et le traitement des matières kératiniques telles que les cheveux, la peau, les cils, les sourcils, les ongles, les lèvres, le cuir chevelu et plus particulièrement les cheveux.

En particulier, les compositions détergentes selon l'invention sont des shampooings, des gels-douche et des bains moussants.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de produits démaquillants.

Les compositions selon l'invention peuvent se présenter sous forme de gel, de lait, de crème, d'émulsion, de lotion épaissie ou de mousse et être utilisées pour la peau, le cuir chevelu, les ongles, les cils, les lèvres et plus particulièrement les cheveux.

Ces compositions détergentes sont de préférence moussantes et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombent dans une éprouvette ayant un diamètre de 50 mm et contenant 50 ml de la composition à tester. La mesure est faite 5 minutes après l'arrêt de l'écoulement de la composition.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage en particulier avec de l'eau.

Ainsi, ce procédé selon l'invention permet le traitement, le soin, le lavage ou le démaquillage de la peau, des cheveux ou de toute autre matière kératinique.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits. Dans les exemples, MA signifie matière active.

### EXEMPLE 1 :

On a réalisé trois compositions de shampooings, l'une conforme à l'invention (composition A) et les 2 autres comparatives (compositions B et C) :

| | A (invention) | B | C |
|---|---|---|---|
| - Sel de triéthanolamine de N-cocoyl glutamine à 30%MA (Foam up doucé GM de KYOWA HAKKO) | 16,7 g (5 gMA) | ― | ― |
| - Cocoylbétaine en solution aqueuse à 30%MA (DEHYTON AB 30 de COGNIS) | 16,7 g (5 gMA) | 16,7 g (5 gMA) | 16,7 g (5 gMA) |
| -Alkyl(C₁₂-C₁₄)éther sulfate de sodium oxyéthyléné à 2,2 moles d'oxyde d'éthylène en solution aqueuse à 26% de MA | 57 g (14,8 gMA) | 57 g (14,8 gMA) | 77 g (20, gMA) |
| N-cocoyl glutamate en solution aqueuse à 30% MA (Acyl glutamate CT12 de AJINOMOTO) | ― | 16,7 g (5 gMA) | ― |
| - Myristate d'isopropyle | 4 g | 4 g | 4 g |
| - Conservateurs | qs | qs | qs |
| Agent de pH qs pH | 6,5 | 6,5 | 6,5 |
| - Eau déminéralisée qsp | 100 g | 100 g | 100 g |

On effectue un shampooing en appliquant environ 1 g de la composition A sur des mèches de 2,5 g de cheveux sensibilisés préalablement mouillés. On fait mousser le shampooing, on laisse pauser pendant 10 minutes puis on rince abondamment à l'eau. On sèche les mèches pendant 10 minutes à 60°C. On fait une deuxième application de la composition.
On procède selon le même mode opératoire que ci-dessus avec les compositions comparatives B et C.
Les experts comparent les mèches deux à deux.

### Composition A/Composition C

Un panel d'experts a évalué l'aspect des cheveux mouillés.
90% des experts indiquent que les cheveux traités avec la composition A selon l'invention sont significativement plus lisses, que ceux traités avec la composition C.

### Composition A/Composition B

Un panel d'experts a évalué l'aspect des cheveux séchés.
90% des experts indiquent que les cheveux traités avec la composition A selon l'invention sont significativement plus lisses et/ou plus doux, que ceux traités avec la composition B.

## Revendications

1. Composition cosmétique détergente, **caractérisée par le fait qu'**elle comprend, dans un milieu cosmétiquement acceptable, au moins un tensioactif anionique choisi parmi les composés N-acylés d'aminoacides polycarboxyliques mono ou poly amidifiés et leurs sels et au moins un agent conditionneur insoluble choisi parmi les huiles de synthèses, les huiles minérales, les huiles végétales, les huiles fluorées ou perfluorées, les cires naturelles ou synthétiques, les composés de type céramide, les esters d'acides carboxyliques choisis parmi les esters de monoalcools et les esters de polyols, les polyols ayant au moins 3 atomes de carbone.

2. Composition selon la revendication 1, **caractérisée par le fait que** ledit tensioactif anionique est choisi parmi les composés N-acylés d'aminoacides polycarboxyliques mono ou poly amidifiés et leurs sels de formule suivante (I) : 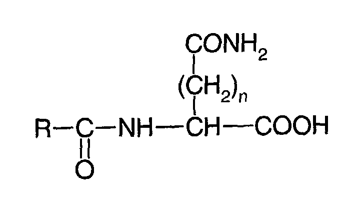 dans laquelle :
R désigne un radical hydrocarboné, saturé ou insaturé, linéaire ou ramifié comportant de 5 à 29 atomes de carbone.
n est égal à 1 ou 2.

3. Composition selon la revendication 2, **caractérisée par le fait que** R désigne un radical alkyle ou alkényle mono ou polyinsaturé comportant de 5 à 29 atomes de carbone et de préférence de 7 à 22 atomes de carbone.

4. Composition selon l'une quelconque des revendications 1 à 3, **caractérisée par le fait que** ledit tensioactif anionique est un sel de N-cocoylglutamine.

5. Composition selon l'une quelconque des revendications précédentes, **caractérisée par le fait que** les huiles de synthèse sont des polyoléfines de type polybutène, hydrogéné ou non, ou de type polydécène, hydrogéné ou non.

6. Composition selon l'une quelconque des revendications 1 à 5, **caractérisée par le fait que** les huiles animales ou végétales sont choisies dans le groupe formé par les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone en particulier alkyle ou alkényle, les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote.

7. Composition selon l'une quelconque des revendications 1 à 6, **caractérisée par le fait que** la cire ou les cires sont choisies parmi la cire de Carnauba, la cire de Candelila, la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis, les cires animales comme les cires d'abeilles, les cires marines, les cires de polyéthylène ou de polyoléfines.

8. Composition selon l'une quelconque des revendications 1 à 7, **caractérisée par le fait que** les composés de type céramides sont choisis parmi ceux répondant à la formule générale (IV): 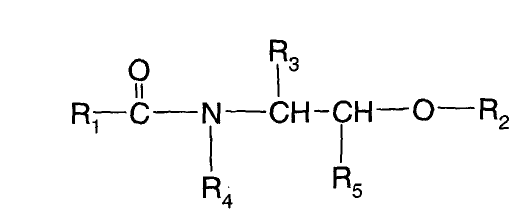 dans laquelle :
- R₁ désigne :
- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifié par un acide gras saturé ou insaturé, linéaire ou ramifié, éventuellement mono ou polyhydroxylé, en C₁-C₃₅;
- soit un radical R"-(NR°-CO)-R', R° désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone va de 9 à 30, R' étant un radical divalent.
- soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, mono ou polyhydroxylé ou non hydroxylé, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ; de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)_{m,} sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

9. Composition selon l'une quelconque des revendications 1 à 8, **caractérisée par le fait que** les composés de type céramides sont choisis parmi :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine
ou les mélanges de ces composés.

10. Composition selon l'une quelconque des revendications 1 à 9, **caractérisée par le fait que** les esters d'acides carboxyliques sont liquides à température inférieure ou égale à 30°C.

11. Composition selon l'une quelconque des revendications 1 à 10, **caractérisée par le fait que** lesdits esters de monoalcools sont choisis parmi les esters d'acides mono, di, tri ou tétracarboxyliques, de préférence en C₂-C₂₆ et de monoalcool, de préférence en C₁-C₂₆.

12. Composition selon l'une quelconque des revendications 1 à 10, **caractérisée par le fait que** lesdits esters de polyols sont choisis parmi les esters d'acides mono di ou tricarboxyliques de préférence en C2-C26 et d'alcools di, tri, tétra ou pentahydroxy en C3-C26.

13. Composition selon l'une quelconque des revendications précédentes, **caractérisée en ce que** les esters d'acides carboxyliques sont choisis parmi les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate de butyle, le stéarate d'isobutyle ; le malate de dioctyle, le laurate d'hexyle, l'isononanate d'isononyle et l'octanoate de cétyle.

14. Composition selon l'une quelconque des revendications 1 à 13, **caractérisée en ce que** le ou les tensioactifs anioniques choisis parmi les composés N-acylés d'aminoacides polycarboxyliques mono ou poly amidifiés sont présents à une concentration allant de 1 à 30 % en poids, de préférence allant de 3 à 15% en poids par rapport au poids total de la composition.

15. Composition selon l'une quelconque des revendications 1 à 14, **caractérisée en ce que** le ou les agents conditionneurs sont présents à une concentration allant de 0,001 % à 10 % en poids par rapport au poids total de la composition, de préférence allant de 0,005 % à 5 % en poids et en particulier allant de 0,01 % à 3 % en poids.

16. Composition selon l'une quelconque des revendications 1 à 15, **caractérisée par le fait qu'**elle comprend en outre au moins un agent tensioactif additionnel choisi parmi les tensioactifs anioniques, cationiques, non ioniques, amphotères et leurs mélanges.

17. Compositions selon la revendication 16, **caractérisées par le fait que** le ou les agents tensioactifs additionnels sont présents à une concentration allant de 0,5% à 40% en poids, de préférence allant de 3% à 30% en poids, et encore plus préférentiellement allant de 5% à 20% en poids, par rapport au poids total de la composition.

18. Composition selon l'une quelconque des revendications 1 à 17, **caractérisée par le fait qu'**elle comprend en outre au moins un additif choisi parmi les épaississants, les parfums, les agents nacrants, les conservateurs, les filtres solaires, les silicones, les polymères anioniques ou non ioniques ou amphotères, les polymères cationiques, les protéines, les hydrolysats de protéines, les acides gras à chaînes linéaires ou ramifiées en C₁₆-C₄₀ tels que l'acide méthyl-18 eicosanoique, les hydroxyacides, les vitamines, les provitamines telles que le panthénol, les agents antipelliculaires et leurs mélanges.

19. Composition selon l'une quelconque des revendications 1 à 18, **caractérisées par le fait qu'**elle se présente sous forme de shampooing, de composition lavantes pour la peau, d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

20. Utilisation d'une composition telle que définie dans l'une quelconque des revendications précédentes pour le lavage des matières kératiniques en particulier les cheveux.

21. Utilisation d'une composition telle que définie dans l'une quelconque des revendications 1 à 19 pour améliorer le démêlage ou le lissage des cheveux , ou pour apporter du volume, de la légèreté, de la douceur, de la souplesse ou de la discipline aux cheveux..

22. Procédé de traitement des matières kératiniques, telles que les cheveux, **caractérisé en ce qu'**il consiste à appliquer sur lesdites matières une composition cosmétique selon l'une des revendications 1 à 19, puis à effectuer éventuellement un rinçage.
